# EUROPEAN PATENT APPLICATION

(11) **EP 4 361 152 A1**
(43) Date of publication of application: **01.05.2024**
(21) Application number: 22827576.4
(22) Date of filing: 21.06.2022
(51) Int. Cl.: C07D 487/04, A61K 31/519, A61P 37/06

(54) **USE OF PYRROLOPYRIMIDINE COMPOUND AND PHARMACEUTICAL COMPOSITION THEREOF FOR TREATING CHRONIC GRAFT VERSUS HOST DISEASE**

(30) Priority: 21.06.2021 CN 202110688144
(71) Applicant: CHIA TAI TIANQING PHARMACEUTICAL GROUP CO., LTD., Lianyungang, Jiangsu 222062 (CN); Shouyao Holdings (Beijing) Co., Ltd., Beijing 100176 (CN); Lianyungang Runzhong Pharmaceutical Co., Ltd., Lianyungang, Jiangsu 222069 (CN)
(72) Inventor: TU, Lifan, Lianyungang Jiangsu 222062 (CN); ZHANG, Xiquan, Lianyungang Jiangsu 222062 (CN); WANG, Xunqiang, Lianyungang Jiangsu 222062 (CN); YU, Ding, Lianyungang Jiangsu 222062 (CN); HUANG, Jianqiang, Lianyungang Jiangsu 222062 (CN)
(74) Representative: Potter Clarkson
(86) International application number: PCT/CN2022/100198
(87) International publication number: WO 2022/268083

(57) **Abstract**

The present invention relates to the use of a pyrrolopyrimidine compound and a pharmaceutical composition thereof for treating chronic graft versus host disease, and particularly relates to the use of a compound of formula (I), a stereoisomer thereof, or a pharmaceutically acceptable salt thereof, and a pharmaceutical composition thereof for treating chronic graft versus host disease.

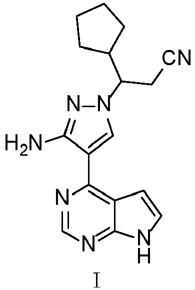

## Description

### CROSS-REFERENCE TO RELATED APPLICATIONS

The present application claims priority and benefit to the Chinese Patent Application No. 202110688144.0 filed with National Intellectual Property Administration, PRC on Jun. 21, 2021, the disclosure of which is incorporated herein by reference in its entirety.

### TECHNICAL FIELD

The present application relates to the field of medicinal chemistry, and relates to use of a pyrrolopyrimidine compound and a pharmaceutical composition thereof for treating chronic graft-versus-host disease, and particularly relates to use of a compound of formula **I,** a stereoisomer thereof, or a pharmaceutically acceptable salt thereof, and a pharmaceutical composition thereof for treating chronic graft-versus-host disease.

### BACKGROUND

Janus kinase (JAK) is a group of non-receptor tyrosine kinases (PTK). It exists in cells and transmits cytokine stimulation signals through the JAK-STAT pathway. The JAK-STAT pathway transmits extracellular chemical signals through the cell membrane to the gene promoter of DNA in the nucleus, which ultimately causes changes in DNA transcription and activity. The JAK-STAT pathway consists of three major components: 1) a receptor; 2) the JAK; and 3) a signal transducer and activator of transcription (STAT) protein. The receptor can be activated by interferons, interleukins, growth factors or other chemical messengers, leading to the autophosphorylation of JAK; the STAT protein binds to the phosphorylated receptor, such that the STAT protein is phosphorylated by JAK; then the phosphorylated STAT protein is separated from the receptor, dimerized and translocated into the nucleus to bind to the specific sites on DNA and alter the transcription (Scott, M. J., C. J. Godshall, et al., (2002) "Jaks, STATs, Cytokines, and Sepsis", Clin Diagn Lab Immunol 9(6): 1153-9).

Allogeneic hematopoietic stem cell transplantation (allo-HSCT) is now widely used for the treatment of hematologic system and non-hematologic system diseases, and is even the only method to cure certain diseases. Graft-versus-host disease (GvHD) is a major complication of allogeneic stem cell transplantation. Because T lymphocytes in the transplanted allogeneic donor graft are stimulated by a series of "cytokine storms" initiated by the recipient, the immune response to the antigen of the recipient is greatly enhanced, and cytotoxic attack is initiated by taking the target cells of the recipient as the target, wherein skin, fiver, and intestinal tract are the main targets.

Chronic graft-versus-host disease (cGVHD) is the most common long-term complication following allogeneic stem cell transplantation (SCT), affecting 30-70% of patients who survive more than the first 100 days. cGVHD and its associated immunodeficiency have been considered to be the major cause of non-relapse mortality (NRM) in allogeneic SCT survivors. Human and animal models have demonstrated that abnormal B-lymphocyte signaling and survival are important in the pathogenesis of cGVHD. B cell targeted drugs, including SYK inhibitors (fostamatinib-Sarantopoulos et al., Biology of Blood and Marrow Transplantation, 21(2015) S11-S18) and BTK inhibitors (ibrutinib-Nakasone et al., Int. J. Hematol. Mar. 27, 2015), are shown to be capable of selectively reducing the function and frequency of abnormal GVHD B cell populations *in vitro.*

### SUMMARY

In one aspect, the present application provides a compound of formula **I,** a stereoisomer thereof or a pharmaceutically acceptable salt thereof for use in treating graft-versus-host disease in a patient:

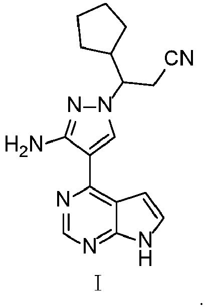

In one aspect, the present application provides a pharmaceutical composition for use in treating graft-versus-host disease in a patient, which comprises the compound of formula **I,** the stereoisomer thereof or the pharmaceutically acceptable salt thereof. In one aspect, the present application provides a method for treating graft-versus-host disease in a patient, which comprises administering to the patient an effective amount of the compound of formula **I,** the stereoisomer thereof or the pharmaceutically acceptable salt thereof, or the pharmaceutical composition thereof described above.

In one aspect, the present application provides use of the compound of formula **I,** the stereoisomer thereof or the pharmaceutically acceptable salt thereof, or the pharmaceutical composition thereof described above for preparing a medicament for treating graft-versus-host disease in a patient.

In one aspect, the present application provides use of the compound of formula **I,** the stereoisomer thereof or the pharmaceutically acceptable salt thereof, or the pharmaceutical composition thereof described above for treating graft-versus-host disease in a patient.

In some embodiments of the present application, the graft-versus-host disease is selected from the group consisting of chronic graft-versus-host disease.

In another aspect, the present application provides a compound of formula **I,** a stereoisomer thereof or a pharmaceutically acceptable salt thereof for use in treating chronic graft-versus-host disease in a patient:

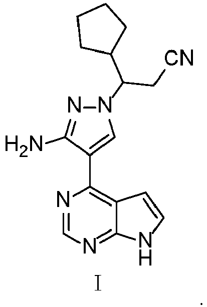

In another aspect, the present application provides a pharmaceutical composition for use in treating chronic graft-versus-host disease in a patient, which comprises the compound of formula **I,** the stereoisomer thereof or the pharmaceutically acceptable salt thereof.

In another aspect, the present application provides a method for treating chronic graft-versus-host disease in a patient, which comprises administering to the patient an effective amount of the compound of formula **I,** the stereoisomer thereof or the pharmaceutically acceptable salt thereof, or the pharmaceutical composition thereof described above.

In another aspect, the present application provides use of the compound of formula **I,** the stereoisomer thereof or the pharmaceutically acceptable salt thereof, or the pharmaceutical composition thereof described above for preparing a medicament for treating chronic graft-versus-host disease in a patient.

In another aspect, the present application provides use of the compound of formula **I,** the stereoisomer thereof or the pharmaceutically acceptable salt thereof, or the pharmaceutical composition thereof described above for treating chronic graft-versus-host disease in a patient.

In some embodiments of the present application, the compound of formula **I,** the stereoisomer thereof or the pharmaceutically acceptable salt thereof described herein is used as a single active agent.

In some embodiments of the present application, the compound of formula **I,** the stereoisomer thereof or the pharmaceutically acceptable salt thereof described herein may be provided in the form of a pharmaceutical composition comprising a therapeutically effective amount of the compound of formula **I,** the stereoisomer thereof or the pharmaceutically acceptable salt thereof.

The pharmaceutical composition of the present application can be prepared by combining the compound of the present application with a suitable pharmaceutically acceptable excipient, and can be formulated, for example, into a solid, semisolid, liquid, or gaseous formulation such as tablet, pill, capsule, powder, granule, ointment, emulsion, suspension, suppository, injection, inhalant, gel, microsphere and aerosol. The pharmaceutical composition of the present application can be manufactured using methods well known in the art, such as conventional mixing, dissolving, granulating, dragee-making, levigating, emulsifying, and lyophilizing. Suitable excipients include, but are not limited to: binders, diluents, wetting agents, disintegrants, lubricants, glidants, sweeteners or flavoring agents, and the like.

In some embodiments of the present application, the pharmaceutical composition is a preparation suitable for oral administration, including tablets, capsules, powders, granules, dripping pills, pastes, pulvis, and the like, preferably tablets and capsules. The oral preparation may be prepared by a conventional method using a pharmaceutically acceptable carrier well known in the art. The pharmaceutically acceptable carrier includes diluents, binders, wetting agents, disintegrants, lubricants, and the like. The diluents include microcrystalline cellulose, mannitol, lactose, sucrose, starch, pregelatinized starch, dextrin, etc., or a mixture thereof; the binders include hydroxypropyl methylcellulose, carboxymethyl cellulose, carboxymethylcellulose sodium, ethyl cellulose, methyl cellulose, hydroxypropyl cellulose, low-substituted hydroxypropyl cellulose, gelatin, polyvinylpyrrolidone, starch, sucrose, glucose, gelatin, etc., or a mixture thereof; the wetting agents include magnesium stearate, talcum powder, polyethylene glycol, sodium dodecyl sulfate, silica gel micropowder, talcum powder, etc., or a mixture thereof; the disintegrants include sodium carboxymethyl starch, dry starch, microcrystalline cellulose, hydroxyethyl methylcellulose, carboxymethylcellulose sodium, carboxymethylcellulose calcium, croscarmellose sodium, low-substituted hydroxypropyl methylcellulose, crospovidone, etc., or a mixture thereof; and the lubricants include magnesium stearate, colloidal silicon dioxide, talcum powder, polyethylene glycol, stearic acid, sodium stearyl fumarate, etc., or a mixture thereof. The pharmaceutical excipients also include coloring agents, sweeteners, coating agents, and the like.

### Compound of Formula I

In some embodiments of the present application, the compound of formula **I** described herein is a compound of formula **II**

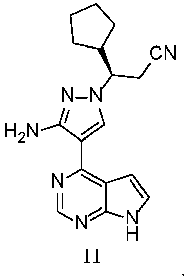

The compound of formula I and the compound of formula II of the present application can be prepared with reference to the preparation methods in Patents No. WO2016095805 and WO2017215627.

### Chronic graft-versus-host disease (cGVHD)

The chronic graft-versus-host disease of the present application refers to a complication caused by allogeneic stem cell transplantation.

In some embodiments of the present application, the chronic graft-versus-host disease is classified into classical chronic graft-versus-host disease and acute and chronic overlap syndrome.

In some embodiments of the present application, the chronic graft-versus-host disease is selected from the group consisting of mild, moderate and severe chronic graft-versus-host diseases (which may be classified according to 2014 NIH Chronic GVHD Diagnosis and Staging Consensus). For the mild chronic graft-versus-host disease, there are 1 or 2 organs or sites involved (except lung) (score ≤ 1); for the moderate, there is at least 1 organ or site involved (score = 2), or 3 or more organs or sites involved (score ≤ 1), or lung involved (score = 1); for the severe, there is any organ or site involved reaching a score of 3 or lung involved (score ≥ 2).

In some embodiments, the chronic graft-versus-host disease described herein is mild chronic graft-versus-host disease. In some embodiments, the chronic graft-versus-host disease is moderate or severe chronic graft-versus-host disease. In some embodiments, the chronic graft-versus-host disease is severe chronic graft-versus-host disease.

In some embodiments of the present application, the organ involved in the chronic graft-versus-host disease includes, but is not limited to, mucosa, skin, oral cavity, eye, gastrointestinal tract, fiver, lung, joint/fascia, and reproductive tract.

In some embodiments, the chronic graft-versus-host disease described herein is refractory and/or dependent chronic graft-versus-host disease. In some embodiments, the chronic graft-versus-host disease described herein is glucocorticoid-refractory and/or dependent chronic graft-versus-host disease.

In some embodiments, the refractory and/or dependent chronic graft-versus-host disease is selected from the group consisting of any one of the following: a) a patient has taken prednisone (or glucocorticoid at the same dose) ≥ 1 mg/kg/day for at least 1 week, and the disease of the patient progresses thereafter or the disease progresses during the period; b) a patient has taken prednisone (or glucocorticoid at the same dose) ≥ 0.5 mg/kg/day or ≥ 1 mg/kg every other day for at least 4 weeks, and the disease symptoms are continuously not improved; or c) the dose of prednisone (or glucocorticoid at the same dose) is increased to > 0.25 mg/kg/day after 2 dose-reduction failures.

In some embodiments, the chronic graft-versus-host disease is refractory and/or dependent moderate or severe chronic graft-versus-host disease. In some embodiments, the chronic graft-versus-host disease is glucocorticoid-refractory and/or dependent moderate or severe chronic graft-versus-host disease.

In some embodiments of the present application, the glucocorticoid is selected from the group consisting of prednisone, meprednisone, prednisone acetate, prednisolone, methylprednisolone, prednisolone acetate, prednisolone sodium succinate, methylprednisolone sodium succinate, betamethasone, beclomethasone propionate, hydrocortisone, dexamethasone, salmeterol/fluticasone, and pulsed high-dose glucocorticoid.

In some embodiments of the present application, the chronic graft-versus-host disease is a chronic graft-versus-host disease that progresses, relapses, is refractory, and/or is dependent after receiving at least one drug therapy. In some embodiments, the chronic graft-versus-host disease is a chronic graft-versus-host disease that progresses, relapses, is refractory, and/or is dependent after receiving at least one hormonal drug therapy. In some embodiments, the chronic graft-versus-host disease is a chronic graft-versus-host disease that progresses, relapses, is refractory, and/or is dependent after receiving glucocorticoid drug therapy.

In some embodiments of the present application, the chronic graft-versus-host disease is a chronic graft-versus-host disease that is intolerable to hormonal drug therapy. In some embodiments, the chronic graft-versus-host disease is a chronic graft-versus-host disease resistant to glucocorticoid. In some embodiments, the chronic graft-versus-host disease is a chronic graft-versus-host disease that has not previously received hormonal drug therapy. In some embodiments, the chronic graft-versus-host disease is a chronic graft-versus-host disease that has not previously received extracorporeal photopheresis therapy. In some embodiments, the chronic graft-versus-host disease is a steroid-refractory chronic graft-versus-host disease.

In some embodiments of the present application, the chronic graft-versus-host disease is a chronic graft-versus-host disease that has previously received one or more lines of systemic treatment for a chronic graft-versus-host disease, wherein the one or more lines of systemic treatment include having previously received extracorporeal photopheresis therapy and/or having previously received drug therapy.

In some embodiments of the present application, the chronic graft-versus-host disease is a chronic graft-versus-host disease that has previously received extracorporeal photopheresis therapy. The extracorporeal photopheresis therapy mainly includes ultraviolet irradiation, wherein the ultraviolet irradiation includes but is not limited to ultraviolet UVA, ultraviolet UVB, ultraviolet UVC and ultraviolet UVD, preferably ultraviolet LJVA.

In some embodiments of the present application, the chronic graft-versus-host disease is a chronic graft-versus-host disease that has previously received drug therapy. In some embodiments of the present application, the drug for the drug therapy includes, but is not limited to, one or more of hormonal drugs, calcineurin inhibitors, mammalian target of rapamycin (M-Tor) inhibitors, and immunosuppressants. The hormonal drug is adrenocortical hormone drug, including but not limited to adrenocorticotropic hormone, glucocorticoid, and mineralocorticoid, preferably glucocorticoid. The hormonal drug includes but is not limited to prednisone, methylprednisone, prednisolone, methylprednisolone, methylprednisolone sodium succinate, betamethasone, beclomethasone propionate, diprospan, hydrocortisone, dexamethasone, salmeterol/fluticasone, or pulsed high-dose glucocorticoid. The calcineurin inhibitor includes but is not limited to tacrolimus or cyclosporine. The mammalian target of rapamycin (M-Tor) inhibitor includes but is not limited to sirolimus, everolimus, temsirolimus, or tacrolimus. The immunosuppressant includes an immune antibody drug, wherein the immune antibody drug includes but is not limited to an anti-T cell monoclonal antibody (anti-CD3 monoclonal antibody), an anti-interleukin-2 receptor antibody, an anti-TNF antibody and the like. The immunosuppressant includes but is not limited to daclizumab, basiliximab, alemtuzumab or rituximab. The drug further includes but is not limited to ganciclovir sodium, imatinib, mycophenolate sodium, mycophenolate mofetil ( CellCept ) , azathioprine, psoralen, methotrexate, hydroxychloroquine, clofazimine, cyclophosphamide, thalidomide, azithromycin, montelukast, sorafenib, ruxolitinib or alefacept.

In some embodiments of the present application, provided is use of the compound of formula **I,** the stereoisomer thereof or the pharmaceutically acceptable salt thereof for preparing a medicament for a chronic graft-versus-host disease that has previously received drug therapy that ended in failure. In some embodiments, provided is use of the compound of formula **I,** the stereoisomer thereof or the pharmaceutically acceptable salt thereof for preparing a medicament for treating a chronic graft-versus-host disease that progresses, relapses, is refractory, and/or is dependent after receiving hormonal drug therapy. In some embodiments, provided is use of the compound of formula **I,** the stereoisomer thereof or the pharmaceutically acceptable salt thereof for preparing a medicament for treating a moderate or severe chronic graft-versus-host disease that progresses, relapses, is refractory, and/or is dependent after receiving hormonal drug therapy. In some embodiments, provided is use of the compound of formula **I,** the stereoisomer thereof or the pharmaceutically acceptable salt thereof for preparing a medicament for treating a chronic graft-versus-host disease that progresses, relapses, is refractory, and/or is dependent after receiving glucocorticoid therapy. In some embodiments, provided is use of the compound of formula **I,** the stereoisomer thereof or the pharmaceutically acceptable salt thereof for preparing a medicament for treating a moderate or severe chronic graft-versus-host disease that progresses, relapses, is refractory, and/or is dependent after receiving glucocorticoid therapy. In some embodiments, provided is use of the compound of formula **I,** the stereoisomer thereof or the pharmaceutically acceptable salt thereof for preparing a medicament for treating a chronic graft-versus-host disease that progresses, relapses, is refractory, and/or is dependent after receiving therapy of one or more of prednisone, meprednisone, prednisone acetate, prednisolone, methylprednisolone, prednisolone acetate, prednisolone sodium succinate, methylprednisolone sodium succinate, salmeterol/fluticasone, cyclosporine, methotrexate, dexamethasone, azithromycin, montelukast, tacrolimus, mycophenolate mofetil, sorafenib and/or ruxolitinib. In some embodiments, provided is use of the compound of formula **I,** the stereoisomer thereof or the pharmaceutically acceptable salt thereof for preparing a medicament for treating a moderate or severe chronic graft-versus-host disease that progresses, relapses, is refractory, and/or is dependent after receiving therapy of one or more of prednisone, methylprednisolone, prednisolone, methylprednisolone sodium succinate, cyclosporine, tacrolimus, mycophenolate mofetil and/or ruxolitinib. In some embodiments, provided is use of the compound of formula **I,** the stereoisomer thereof or the pharmaceutically acceptable salt thereof for preparing a medicament for treating a moderate or severe chronic graft-versus-host disease that progresses, relapses, is refractory, and/or is dependent after receiving therapy of one or more of prednisone, cyclosporine, tacrolimus, mycophenolate mofetil and/or ruxolitinib.

In some embodiments, provided is use of the compound of formula **I,** the stereoisomer thereof or the pharmaceutically acceptable salt thereof for preparing a medicament for treating a moderate or severe chronic graft-versus-host disease that progresses, relapses, is refractory, and/or is dependent after receiving prednisone and cyclosporine therapy. In some embodiments, provided is use of the compound of formula **I,** the stereoisomer thereof or the pharmaceutically acceptable salt thereof for preparing a medicament for treating a moderate or severe chronic graft-versus-host disease that progresses, relapses, is refractory, and/or is dependent after receiving combination treatment of tacrolimus, mycophenolate mofetil, methotrexate, and dexamethasone. In some embodiments, provided is use of the compound of formula **I,** the stereoisomer thereof or the pharmaceutically acceptable salt thereof for preparing a medicament for treating a moderate or severe chronic graft-versus-host disease that progresses, relapses, is refractory, and/or is dependent after receiving combination treatment of tacrolimus, mycophenolate mofetil, prednisone, and sorafenib. In some embodiments, provided is use of the compound of formula **I,** the stereoisomer thereof or the pharmaceutically acceptable salt thereof for preparing a medicament for treating a moderate or severe chronic graft-versus-host disease that progresses, relapses, is refractory, and/or is dependent after receiving combination treatment of tacrolimus, prednisone, and mycophenolate mofetil.

### Administration regimen

In some embodiments of the present application, the administration cycle for treating chronic graft-versus-host disease in a patient is 2-6 weeks. In some embodiments of the present application, the administration cycle for treating chronic graft-versus-host disease in a patient is 2 weeks, 3 weeks, 4 weeks, 5 weeks, 6 weeks, or a range formed by any of the aforementioned values, or any value within the range. In some embodiments of the present application, the administration cycle for treating chronic graft-versus-host disease in a patient is 4 weeks.

The amount of the compound, the stereoisomer thereof or the pharmaceutically acceptable salt thereof disclosed herein can be determined on the basis of severity of the disease, response, any treatment-related toxicity, and the age and health of the patient. For example, it can be determined on the basis of the subject/patient's blood routine examination results, which include platelet count, neutrophil count, hemoglobin concentration, etc. In some embodiments, a daily dose of the compound, the stereoisomer thereof or the pharmaceutically acceptable salt thereof disclosed herein administered is 1 mg to 50 mg. In some embodiments, a daily dose of the compound, the stereoisomer thereof or the pharmaceutically acceptable salt thereof disclosed herein administered may be selected from the group consisting of 1 mg, 5 mg, 10 mg, 15 mg, 20 mg, 25 mg, 30 mg, 35 mg, 40 mg, 45 mg, or 50 mg, or a range of any two of the foregoing values as endpoints or any value within the range, for example, 1 mg to 50 mg, 5 mg to 50 mg, 5 mg to 40 mg, 5 mg to 30 mg, 5 mg to 25 mg, 5 mg to 20 mg, or 10 mg to 20 mg. In some specific embodiments, a daily dose of the compound, the stereoisomer thereof or the pharmaceutically acceptable salt thereof disclosed herein administered may be selected from the group consisting of 5 mg to 30 mg, 5 mg to 25 mg, 5 mg to 20 mg, or 10 mg to 20 mg. In some specific embodiments, a daily dose of the compound, the stereoisomer thereof or the pharmaceutically acceptable salt thereof disclosed herein administered may be selected from the group consisting of 1 mg, 2 mg, 5 mg, 8 mg, 10 mg, 12 mg, 15 mg, 18 mg, 20 mg, 22 mg, 25 mg, 28 mg, 30 mg, 32 mg, 35 mg, 38 mg, 40 mg, 42 mg, 45 mg, 48 mg, or 50 mg, or a range of any two of the foregoing values as endpoints or any value within the range, for example, 5 mg to 30 mg, 5 mg to 25 mg, 5 mg to 20 mg, or 10 mg to 20 mg.

In some embodiments of the present application, a daily dose of the compound, the stereoisomer thereof or the pharmaceutically acceptable salt thereof disclosed herein is 1 mg to 50 mg. In some embodiments, a daily dose of the compound, the stereoisomer thereof or the pharmaceutically acceptable salt thereof disclosed herein is 1 mg, 5 mg, 10 mg, 15 mg, 20 mg, 25 mg, 30 mg, 35 mg, 40 mg, 45 mg, or 50 mg, or a range of any two of the foregoing values as endpoints or any value within the range, for example, 1 mg to 50 mg, 5 mg to 50 mg, 5 mg to 40 mg, 5 mg to 30 mg, 5 mg to 25 mg, 5 mg to 20 mg, or 10 mg to 20 mg. In some specific embodiments, a daily dose of the compound, the stereoisomer thereof or the pharmaceutically acceptable salt thereof disclosed herein is 5 mg to 30 mg, 5 mg to 25 mg, 5 mg to 20 mg, or 10 mg to 20 mg. In some specific embodiments, a daily dose of the compound, the stereoisomer thereof or the pharmaceutically acceptable salt thereof disclosed herein is 1 mg, 2 mg, 5 mg, 8 mg, 10 mg, 12 mg, 15 mg, 18 mg, 20 mg, 22 mg, 25 mg, 28 mg, 30 mg, 32 mg, 35 mg, 38 mg, 40 mg, 42 mg, 45 mg, 48 mg, or 50 mg, or a range of any two of the foregoing values as endpoints or any value within the range, for example, 5 mg to 30 mg, 5 mg to 25 mg, 5 mg to 20 mg, or 10 mg to 20 mg.

The compound, the stereoisomer thereof or the pharmaceutically acceptable salt thereof disclosed herein may be administered once or multiple times a day. In some embodiments, the compound, the stereoisomer thereof or the pharmaceutically acceptable salt thereof disclosed herein is administered once or twice a day. The compound, the stereoisomer thereof or the pharmaceutically acceptable salt thereof disclosed herein may also be administered in a single-dose. In one embodiment, the compound, the stereoisomer thereof or the pharmaceutically acceptable salt thereof disclosed herein is administered in a single-dose once or twice a day. In one embodiment, the compound, the stereoisomer thereof or the pharmaceutically acceptable salt thereof disclosed herein is administered in the form of a single-dose oral solid formulation once or twice a day. In one specific embodiment, the compound, the stereoisomer thereof or the pharmaceutically acceptable salt thereof disclosed herein is administered in the form of a single-dose oral solid formulation twice a day.

The compound, the stereoisomer thereof or the pharmaceutically acceptable salt thereof disclosed herein may also be administered in a multiple-dose. In one embodiment, the compound, the stereoisomer thereof or the pharmaceutically acceptable salt thereof disclosed herein is administered in a multiple-dose once or twice a day. In one embodiment, the compound, the stereoisomer thereof or the pharmaceutically acceptable salt thereof disclosed herein is administered in the form of a multiple-dose oral solid formulation once or twice a day. In one specific embodiment, the compound, the stereoisomer thereof or the pharmaceutically acceptable salt thereof disclosed herein is administered in the form of a multiple-dose oral solid formulation twice a day. In one specific embodiment, the compound, the stereoisomer thereof or the pharmaceutically acceptable salt thereof disclosed herein is orally administered in the form of a multiple-dose oral solid formulation twice a day at fasting.

In some embodiments of the present application, the compound of formula **I,** the stereoisomer thereof or the pharmaceutically acceptable salt thereof is provided in the form of a pharmaceutical composition, preferably a single-dose pharmaceutical composition. In some embodiments, the pharmaceutical composition comprises 1 mg to 50 mg of the compound, the stereoisomer thereof or the pharmaceutically acceptable salt thereof disclosed herein. In some embodiments, the pharmaceutical composition comprises 1 mg, 2 mg, 5 mg, 8 mg, 10 mg, 12 mg, 15 mg, 18 mg, 20 mg, 22 mg, 25 mg, 28 mg, 30 mg, 32 mg, 35 mg, 38 mg, 40 mg, 42 mg, 45 mg, 48 mg or 50 mg, or a range of any two of the foregoing values as endpoints or any value within the range of the compound, the stereoisomer thereof or the pharmaceutically acceptable salt thereof disclosed herein, for example, 5 mg to 30 mg, 5 mg to 25 mg, 5 mg to 20 mg, or 10 mg to 20 mg.

In some embodiments of the present application, the pharmaceutical composition of the compound of formula **I,** the stereoisomer thereof or the pharmaceutically acceptable salt thereof is in a daily dose. In some embodiments of the present application, the pharmaceutical composition of the compound of formula **I,** the stereoisomer thereof or the pharmaceutically acceptable salt thereof is in twice-daily doses. In some embodiments of the present application, the twice-daily doses are the same.

In some embodiments of the present application, the pharmaceutical composition of the compound of formula **I,** the stereoisomer thereof or the pharmaceutically acceptable salt thereof is in twice-daily doses, with each dose being a single-dose or a multiple-dose.

In some embodiments of the present application, the pharmaceutical composition of the compound of formula **I,** the stereoisomer thereof or the pharmaceutically acceptable salt thereof is in twice-daily doses, with each dose being a multiple-dose consisting of single-doses of 5 mg, 10 mg, 15 mg and/or 20 mg of the compound of formula **I,** the stereoisomer thereof or the pharmaceutically acceptable salt thereof. In some embodiments of the present application, the pharmaceutical composition consists of single-doses of 5 mg of the compound of formula **I,** the stereoisomer thereof or the pharmaceutically acceptable salt thereof.

In some embodiments of the present application, the pharmaceutical composition of the compound of formula **I,** the stereoisomer thereof or the pharmaceutically acceptable salt thereof is in twice-daily doses, with each dose being a single-dose of 5 mg, 10 mg, 15 mg and/or 20 mg of the compound of formula **I,** the stereoisomer thereof or the pharmaceutically acceptable salt thereof. In some embodiments of the present application, the single-dose is 20 mg of the compound of formula **I,** the stereoisomer thereof or the pharmaceutically acceptable salt thereof.

In some embodiments of the present application, the pharmaceutical composition of the compound of formula **I,** the stereoisomer thereof or the pharmaceutically acceptable salt thereof is in twice-daily doses, with each dose being a multiple-dose of 10 mg or 15 mg of the compound of formula **I,** the stereoisomer thereof or the pharmaceutically acceptable salt thereof.

In some embodiments of the present application, the pharmaceutical composition of the compound of formula **I,** the stereoisomer thereof or the pharmaceutically acceptable salt thereof is packaged in a kit, which also contains instructions for use of the compound of formula **I,** the stereoisomer thereof or the pharmaceutically acceptable salt thereof in treating chronic graft-versus-host disease.

In some embodiments of the present application, in the method or use for treating chronic graft-versus-host disease, the pharmaceutical composition of the compound of formula **I,** the stereoisomer thereof or the pharmaceutically acceptable salt thereof is in a daily dose and is administered as follows: the pharmaceutical composition of the compound of formula **I,** the stereoisomer thereof or the pharmaceutically acceptable salt thereof is administered once or twice a day; in some embodiments of the present application, the pharmaceutical composition of the compound of formula **I,** the stereoisomer thereof or the pharmaceutically acceptable salt thereof is administered twice a day in the same dose; in some embodiments of the present application, the pharmaceutical composition of the compound of formula **I,** the stereoisomer thereof or the pharmaceutically acceptable salt thereof is administered twice a day in the same dose at an interval of 12 h.

In some embodiments of the present application, 28 days constitute one treatment cycle, in which the pharmaceutical composition of the compound of formula **I,** the stereoisomer thereof or the pharmaceutically acceptable salt thereof is administered consecutively from day 1 to day 28.

In some embodiments of the present application, 28 days constitute one treatment cycle, in which the pharmaceutical composition of the compound of formula **I,** the stereoisomer thereof or the pharmaceutically acceptable salt thereof is administered daily consecutively from day 1 to day 28.

In some embodiments of the present application, 28 days constitute one treatment cycle, in which the pharmaceutical composition of the compound of formula **I,** the stereoisomer thereof or the pharmaceutically acceptable salt thereof is administered twice a day consecutively from day 1 to day 28.

In some embodiments of the present application, 28 days constitute one treatment cycle, in which the pharmaceutical composition of the compound of formula **I,** the stereoisomer thereof or the pharmaceutically acceptable salt thereof is administered twice a day at fasting consecutively from day 1 to day 28.

In some embodiments of the present application, 28 days constitute one treatment cycle, in which the pharmaceutical composition of the compound of formula **I,** the stereoisomer thereof or the pharmaceutically acceptable salt thereof is administered twice a day consecutively from day 1 to day 28. Each dose of the pharmaceutical composition of the compound of formula **I,** the stereoisomer thereof or the pharmaceutically acceptable salt thereof is a single-dose of 5 mg of the compound of formula **I,** the stereoisomer thereof or the pharmaceutically acceptable salt thereof.

In some embodiments of the present application, 28 days constitute one treatment cycle, in which the pharmaceutical composition of the compound of formula **I,** the stereoisomer thereof or the pharmaceutically acceptable salt thereof is administered twice a day consecutively from day 1 to day 28. Each dose of the pharmaceutical composition of the compound of formula **I,** the stereoisomer thereof or the pharmaceutically acceptable salt thereof is a multiple-dose of 10 mg or 15 mg of the compound of formula **I,** the stereoisomer thereof or the pharmaceutically acceptable salt thereof.

In some embodiments of the present application, the pharmaceutical composition of the compound of formula **I,** the stereoisomer thereof or the pharmaceutically acceptable salt thereof is packaged in a kit, which contains 1-day dose, 2-day dose, 3-day dose, 4-day dose, 5-day dose, 6-day dose, 7-day dose, 8-day dose, 9-day dose, 10-day dose, 11-day dose, 12-day dose, 13-day dose, 14-day dose, 15-day dose, 16-day dose, 17-day dose, 18-day dose, 19-day dose, 20-day dose, 21-day dose, 22-day dose, 23-day dose, 24-day dose, 25-day dose, 26-day dose, 27-day dose, or 28-day dose, or a range of any two of the foregoing values as endpoints of the compound of formula **I,** the stereoisomer thereof or the pharmaceutically acceptable salt thereof, or the pharmaceutical composition thereof described above. In some embodiments of the present application, the pharmaceutical composition of the compound of formula **I,** the stereoisomer thereof or the pharmaceutically acceptable salt thereof described above is packaged in a kit, which contains 1-day dose, 2-day dose, 3-day dose, 4-day dose, 5-day dose, 6-day dose, 7-day dose, 14-day dose, 21-day dose, or 28-day dose, or a range of any two of the foregoing values as endpoints of the compound of formula **I,** the stereoisomer thereof or the pharmaceutically acceptable salt thereof, or the pharmaceutical composition thereof described above.

In some embodiments of the present application, 28 days constitute one treatment cycle, in which a total dose of 140-840 mg of the pharmaceutical composition of the compound of formula **I,** the stereoisomer thereof or the pharmaceutically acceptable salt thereof (calculated based on the active ingredient, the compound of formula (**I**)) is administered. In some embodiments of the present application, the total dose of the pharmaceutical composition of the compound of formula **I,** the stereoisomer thereof or the pharmaceutically acceptable salt thereof is selected from the group consisting of 140 mg, 280 mg, 420 mg, 560 mg, 700 mg and 840 mg or from a range formed by any two of the aforementioned values (calculated based on the active ingredient, the compound of formula (**I**)). In some embodiments of the present application, the total dose of the pharmaceutical composition of the compound of formula **I,** the stereoisomer thereof or the pharmaceutically acceptable salt thereof is preferably 280-840 mg (calculated based on the active ingredient, the compound of formula (**I**)). In some embodiments of the present application, the total dose of the pharmaceutical composition of the compound of formula **I,** the stereoisomer thereof or the pharmaceutically acceptable salt thereof is preferably 280 mg, 560 mg, or 840 mg (calculated based on the active ingredient, the compound of formula (**I**)).

In some embodiments of the present application, the treatment cycle is repeated as long as the disease is still under control and the administration regimen is clinically tolerable.

The compound, the stereoisomer thereof or the pharmaceutically acceptable salt thereof disclosed herein can be administered by various routes, including but not limited to: oral, parenteral, intraperitoneal, intravenous, intra-arterial, transdermal, sublingual, intramuscular, rectal, transbuccal, intranasal, inhalational, vaginal, intraocular, topical, subcutaneous, intraadipose, intra-articular and intrathecal administrations. In one specific embodiment, the route is oral administration.

In some embodiments of the present application, the pharmaceutical composition of the compound of formula I, the stereoisomer thereof or the pharmaceutically acceptable salt thereof may be formulated in a form suitable for oral administration to a human, for example, including but not limited to tablet, pill, capsule, powder and granule.

In some embodiments of the present application, the pharmaceutical composition of the compound of formula I, the stereoisomer thereof or the pharmaceutically acceptable salt thereof is in oral tablets.

In some embodiments of the present application, a single-dose of the oral tablet of the compound of formula **I,** the stereoisomer thereof or the pharmaceutically acceptable salt thereof is 5 mg or 20 mg.

The administration regimen described herein is also suitable for graft-versus-host disease.

### Technical Effects

The compound of formula **I,** the stereoisomer thereof or the pharmaceutically acceptable salt thereof, or the pharmaceutical composition thereof disclosed herein has favorable therapeutic effects, including but not limited to, better objective response rate, complete response rate, and overall survival, longer duration of response (DOR), higher failure-free survival, and lower non-relapse mortality and original disease relapse/progression events. The compound of formula **I,** the stereoisomer thereof or the pharmaceutically acceptable salt thereof, or the pharmaceutical composition thereof disclosed herein also has favorable safety in addition to favorable therapeutic effects, including but not limited to a lower incidence of adverse events.

### Definitions and Explanations

Unless otherwise stated, the following terms used herein shall have the following meanings. A certain term, unless otherwise specifically defined, should not be considered uncertain or unclear, but construed according to its common meaning in the field. When referring to a trade name, it is intended to refer to its corresponding commercial product or its active ingredient.

As used herein, unless otherwise stated, the terms "comprise", "comprises" and "comprising" or equivalents thereof are openended statements and mean that elements, components and steps that are not specified may be included in addition to those listed.

All patents, patent applications and other identified publications are explicitly incorporated herein by reference for the purpose of description and disclosure. Any reference to these publications herein shall not be construed as an admission that the publications form part of the commonly recognized knowledge in the art.

The term "pharmaceutically acceptable" is used herein for those compounds, materials, compositions, and/or dosage forms which are, within the scope of sound medical judgment, suitable for use in contact with the tissues of human beings and animals without excessive toxicity, irritation, allergic response, or other problems or complications, and commensurate with a reasonable benefit/risk ratio.

The term "pharmaceutically acceptable salt" includes salts formed from basic radicals and free acids and salts formed from acidic radicals and free bases. The pharmaceutically acceptable salt described herein is selected from the group consisting of maleate salt, hydrochloride salt, hydrobromide salt, sulfate salt, phosphate salt, nitrate salt, acetate salt, lactate salt, malonate salt, succinate salt, fumarate salt, malate salt, mandelate salt, tartrate salt, citrate salt, ascorbate salt, palmitate salt, benzoate salt, phenylacetate salt, cinnamate salt, salicylate salt, methanesulfonate salt, benzenesulfonate salt and methylbenzenesulfonate salt.

As used herein, the amount of the compound of formula I, e.g., the amount administered, the dose or the amount in the pharmaceutical composition, is calculated based on its free base form.

As used herein, if the compound in the pharmaceutical combination has, for example, at least one basic site, an acid addition salt may be formed. If needed, it may further form an acid addition salt with additional existing basic sites. A compound with at least one acidic group (for example, -COOH) can further form a salt with a base. A compound, for example, comprising both carboxyl and amino, can further form an inner salt.

The compound of the present application can be asymmetrical, for example, has one or more stereoisomers. Unless otherwise stated, all stereoisomers are included, for example, enantiomers and diastereoisomers. The compound with asymetrical carbon atoms of the present application can be separated in an optically pure form or in a racemic form. The optically pure form can be separated from a racemic mixture or can be synthesized using a chiral raw material or a chiral reagent.

The term "patient" includes a mammal, such as a human, dog, cow, horse, pig, sheep, goat, cat, mouse, rabbit, rat or transgenic non-human animal. In some embodiments, the patient is a human.

The term "pharmaceutical composition" refers to a mixture consisting of one or more of the compounds or the salts thereof, or the pharmaceutical combinations thereof disclosed herein and a pharmaceutically acceptable excipient. The pharmaceutical composition is intended to facilitate the administration of the compound or the pharmaceutical combination thereof of the present application to a subject.

The term "treatment" generally refers to obtaining desired pharmacological and/or physiological effects, including partially or completely stabilizing or curing a disease and/or an effect that the disease has. As used herein, "treat", "treating" or "treatment" encompasses any treatment of a disease in a patient, including (a) inhibiting a symptom of the disease, i.e., blocking the progression of the disease; or (b) alleviating a symptom of the disease, i.e., causing remission of the disease or the symptom.

The term "effective amount" refers to an amount of the compound of the present application for (i) treating a specific disease, condition or disorder, or (ii) alleviating, ameliorating or eliminating one or more symptoms of a specific disease, condition or disorder. The amount of the compound of the present application composing the "therapeutically effective amount" varies depending on the compound, the disease state and its severity, the administration regimen, and the age of the mammal to be treated, but can be determined routinely by those skilled in the art in accordance with their knowledge and the present disclosure.

The term "single dose" refers to the smallest unit of packaging containing a certain quantity of pharmaceutical product; for example, in a box of seven capsules, each capsule is a single dose; or a vial of injection is a single dose.

The term "multiple doses" consists of multiple single doses.

The terms "administer", "administration" and "administering" refer to physically introducing the composition comprising a therapeutic agent to an individual using any of a variety of methods and delivery systems known to those skilled in the art. In certain embodiments, the administration is oral administration.

The term "daily dose" refers to a dose administered to a patient per day.

The term "cGVHD" refers to chronic graft-versus-host disease.

The term "bid" refers to administrating twice a day.

The terms "day", "daily", etc., when referred to in an administration regimen, refer to the time within a calendar day that starts at midnight and ends at the next midnight.

As used herein, 2014 NIH cGVHD meeting consensus criteria was used for clinical diagnosis, disease grading, and evaluation of disease status or effectiveness.

As used herein, the severity of adverse events was determined using the NCI-CTC AE 5.0 criteria.

### DETAILED DESCRIPTION

The present invention will be illustrated in more detail through specific examples. The following examples are provided for illustrative purposes only, and are not intended to limit the present invention in any way.

### Preparation Example 1: Preparation of Solid Pharmaceutical Composition Tablets Containing 5 mg and 20 mg of Active Ingredient

The formula for solid pharmaceutical composition tablets containing 5 mg and 20 mg of the active ingredient is shown in Table 1.

**Table 1. Formula for 5 mg and 20 mg tablets**

| **Composition** | **Formulation (mg)** | |
|---|---|---|
| Compound of formula **II** | 5 | 20 |
| Mannitol | 35.275 | 141.1 |
| Microcrystalline cellulose | 70 | 280 |
| Croscarmellose sodium | 3.6 | 14.4 |
| Sodium dodecyl sulfate | 0.125 | 0.5 |
| Hydroxypropyl cellulose | 4.8 | 19.2 |
| Magnesium stearate | 1.2 | 4.8 |
| Purified water | Proper amount | Proper amount |

### Procedures:

1) Mannitol, microcrystalline cellulose and croscarmellose sodium were mixed to prepare a mixture A for further use; Preparation of drug substance suspension: Hydroxypropyl cellulose was dissolved in purified water to prepare a 4% (w/w) hydroxypropyl cellulose solution; sodium dodecyl sulfate was dissolved; the compound of formula II was added and dispersed by stirring to prepare a drug substance suspension;
2) Fluidized bed granulation and drying: the drug substance suspension was applied to the mixture A by spraying for fluidized granulation; Granulation parameters: inlet air temperature: 55-80 °C, spraying pressure: 600-1000 mbar, material temperature: 25-35 °C. Drying started after the spraying, and ended when the material temperature was higher than 45 °C. The materials were sized in a mill through a sieve with a mesh size of Φ 0.6-1.2 mm, and dried granules were obtained;
3) The dried granules and magnesium stearate were sequentially fed into a hopper mixer and well mixed to give a solid pharmaceutical composition for tableting.

### Example 1 Clinical Protocol for Chronic Graft-Versus-Host Disease

The subjects of the study were glucocorticoid-refractory/dependent moderate and severe cGVHD subjects. The safety and the tolerability of the tablets of the compound of formula **I** in the glucocorticoid-refractory/dependent moderate and severe cGVHD subjects were evaluated, and the efficacy and the safety of the tablets of the compound of formula **I** in the glucocorticoid-refractory/dependent moderate and severe cGVHD subjects were further evaluated.

### 1.1. Administration regimen

Method of administration: orally administered twice daily at fasting, with 28 consecutive days of administration as one treatment cycle.

Drug: the tablets of the compound of formula **I** with 5 mg or 20 mg of the active ingredient, the compound of formula I being its stereoisomer, a compound of formula **II**

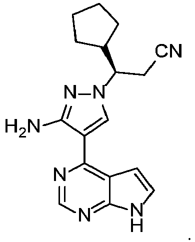

### 1.2. Enrollment criteria

1) Age ≥ 12 years; KPS score ≥ 60; an expected survival time of more than 6 months;
2) Previously received allogeneic hematopoietic stem cell transplantation;
3) Clinically diagnosed as moderate or severe cGVHD according to NIH criteria, defined as follows
   a) moderate cGVHD: ≥ 1 organ (except lung) with score of 2; or at least 3 organs with score ≤ 1; or a lung score of 1;
   b) severe cGVHD: any organ score ≥ 3 or a lung score ≥ 2;
4) cGVHD diagnosed as glucocorticoid-refractory and/or dependent according to NIH criteria that satisfied any one of the following conditions:
   a) a patient had taken prednisone (or glucocorticoid at the same dose) ≥ 1 mg/kg/day for at least 1 week, and the disease progressed thereafter or the disease progressed during the period;
   b) a patient had taken prednisone (or glucocorticoid at the same dose) ≥ 0.5 mg/kg/day or ≥ 1 mg/kg every other day for at least 4 weeks, and the disease symptoms were continuously not improved;
   c) the dose of prednisone (or glucocorticoid at the same dose) was increased to > 0.25 mg/kg/day after 2 dose-reduction failures;
5) Previously received one or more lines of systemic treatment for cGVHD;
6) Laboratory test values need to meet:
   a) liver and kidney functions: serum creatinine ≤ 1.5 × ULN; AST, ALT and alkaline phosphatase ≤ 3 × ULN; total bilirubin ≤ 2 × ULN; creatinine clearance rate ≥ 30 mL/min (according to Cockcroft-Gault formula); (subjects with liver involved did not need to meet the requirements for AST, ALT, alkaline phosphatase, and total bilirubin)
   b) Blood function (no bleeding and no growth factor used within 7 days): ANC ≥ 1.0 × 10⁹/L; PLT ≥ 30 × 10⁹/L; hemoglobin ≥ 80 g/L;
   c) Blood coagulation: PT/INR < 1.5 × ULN, PTT < 1.5 × ULN;
7) Voluntary participation, written informed consent and good compliance.

### 1.3. Evaluation method

### NIH-based cGVHD response criteria

During the study, the investigator would evaluate the cGVHD activity through NIH consensus on cGVHD to define the response effect and make a record accordingly.

Complete response (CR): all reversible clinical symptoms of cGVHD were completely alleviated. Irreversible clinical manifestations would be defined by NIH consensus criteria.

Partial response (PR): there was improvement in at least one organ or site, but no improvement in all other organs or sites. Stable disease (SD): cGVHD clinical performance was not worsened compared to baseline.

Disease progression (PD): any one organ or site was worsened. In addition, the emergence of new clinical manifestations of cGVHD was also counted as progression.

### cGVHD symptom score

The change of score of the Lee cGVHD symptom scale ≥ 7 would be considered clinically significant and would be associated with an improvement in quality of life.

### Failure-free survival (FFS)

Failure-free survival was defined as the percentage of subjects without death and recurrence of the primary malignant tumor and with no new immunosuppressive therapy for GVHD.

### 1.4. Evaluation index

The evaluation index may be selected from the group consisting of:
Objective response rate (ORR): refers to the percentage of subjects in complete response (CR) or partial response (PR) as determined by the investigator according to NIH cGVHD response assessment.

Best objective response rate (ORR): refers to the percentage of subjects whose best response was complete response (CR) or partial response (PR) as determined by the investigator according to NIH cGVHD response assessment.

Duration of response (DOR): defined as the date from the first recording of response to the first recording of disease progression, death or the start of any new systemic treatment for cGVHD.

Failure-free survival (FFS): the percentage of subjects without death and recurrence of the malignant tumor and with no new immunosuppressive therapy for GVHD.

Glucocorticoid use change: glucocorticoid use would be monitored during the study. A reduction in glucocorticoid demand would be considered as evidence of the effectiveness of the test drug.

Change in cGVHD symptom scale: the symptom scale of the subject was improved. Symptom changes would be measured according to the Lee cGVHD symptom scale. The change of score of the Lee cGVHD symptom scale ≥ 7 would be considered a significant improvement and would be associated with an improvement in quality of life.

Overall survival (OS): refers to the time from the start of the first administration to death due to various causes. Non-relapse mortality (NRM): defined as the date from the first administration to the date of relapse/progression death of the non-hematologic system disease.

### 1.5 Results of trial

### 1.5.1 Safety

Tablets of the compound of formula **I** show good tolerability and safety, with overall controlled adverse events.

### 1.5.2 Efficacy

Of the patients evaluated in Table 2, 1 patient was in complete response (CR) and 7 patients were in partial response (PR). The compound of formula I of the present application were found to have clinical benefits in the treatment of chronic graft-versus-host disease.

**Table 2. Evaluation of patient effectiveness**

| Subject No. | History of previous therapy | Dosage regimen | Administration cycle | C0 organ score | Efficacy evaluation | | | | |
|---|---|---|---|---|---|---|---|---|---|
| | | | | | C1 organ score and efficacy | C3 organ score and efficacy | C6 organ score and efficacy | C9 organ score and efficacy | C12 organ score and efficacy |
| 1 | Cyclosporine soft capsule + prednisone acetate tablet | 10mg bid | C13 | 3 points for skin, 1 point for eye, 2 points for oral cavity, 2 points for esophagus, 2 points for upper digestive tract, 3 points for joint fascia, 3 points for lung (35.4%), comprehensive score 7 | 3 points for skin, 1 point for eye, 1 point for oral cavity, 2 points for esophagus, 1 point for upper digestive tract, 3 points for lung (34.4%), 2 points for joint fascia, comprehensive score 6; efficacy: PR | 2 points for skin, 0 points for eye, 1 point for oral cavity, 1 point for esophagus, 0 points for upper digestive tract, 3 points for lung (31%), 2 points for joint fascia, comprehensive score 5; efficacy: PR | 2 points for skin, 0 points for eye, 1 point for oral cavity, 1 point for esophagus, 0 points for upper digestive tract, 3 points for lung (33%), 2 points for joint fascia, comprehensive score 5; efficacy: PR | 2 points for skin, 0 points for eye, 1 point for oral cavity, 1 point for esophagus, 0 points for upper digestive tract, 3 points for lung (35.1%), 2 points for joint fascia, comprehensive score 5; efficacy: PR | 2 points for skin, 0 points for eye, 1 point for oral cavity, 1 point for esophagus, 0 points for upper digestive tract, 3 points for lung (35.1%), 2 points for joint fascia, comprehensive score 5; efficacy: PR |
| 2 | Cyclosporine soft capsule + prednisone acetate tablet | 10mg bid | C11 | 2 points for skin, 1 point for eye, comprehensive score 3 | 1 point for skin, 1 point for eye, comprehensive score 1; efficacy: PR | 1 point for skin, 1 point for eye, comprehensive score 1; efficacy: PR | 1 point for skin, 1 point for eye, comprehensive score 1; efficacy: PR | 0 points for skin, 0 points for eye, comprehensive score 0; efficacy: CR | 0 points for skin, 0 points for eye, comprehensive score 0; efficacy: CR |
| 3 | Cyclosporine soft capsule + prednisone acetate tablet | 10mg bid | C11 | 1 point for oral cavity, 1 point for eye, 1 point for esophagus, 1 point for lung (75%), 1 point for joint, comprehensive score 3 | 1 point for oral cavity, 1 point for eye, 1 point for esophagus, 0 points for lung (81.8%), 1 point for joint, comprehensive score 2; efficacy: PR | 1 point for oral cavity, 1 point for eye, 1 point for esophagus, 0 points for lung (81.0%), 1 point for joint, comprehensive score 1; efficacy: PR | 1 point for oral cavity, 1 point for eye, 1 point for esophagus, 0 points for lung (80%), 1 point for joint, comprehensive score 1; efficacy: PR | 0 points for oral cavity, 1 point for eye, 0 points for esophagus, 0 points for lung, 1 point for joint, comprehensive score 1; efficacy: PR | - |
| 4 | Cyclosporine soft capsule + | 10mg bid | C10 | 2 points for eye, 3 points for lung | 1 point for eye, 3 points for lung (25%), 1 | 1 point for eye, 3 points for lung (28.1%), 1 | 1 point for eye, 3 points for lung (25.0%), 1 | 1 point for eye, 3 points for lung (26.6%), 1 | - |
| | prednisone acetate tablet | | | (24.7%), 1 point for joint, comprehensive score 7 | point for joint, comprehensive score 6; efficacy: PR | point for joint, comprehensive score 5; efficacy: PR | point for joint, comprehensive score 5; efficacy: PR | point for joint, comprehensive score 5; efficacy: PR | |
| 5 | Tacrolimus + mycophenolate mofetil + methotrexate + dexamethasone | 15mg bid | C10 | 2 points for skin, 2 points for eye, 1 point for oral cavity, 1 point for lower digestive tract, 3 points for lung (17.7%), 1 point for joint fascia, comprehensive score 8 | 2 points for skin, 1 point for eye, 1 point for oral cavity, 1 point for lower digestive tract, 3 points for lung (20.5%), 1 point for joint fascia, comprehensive score 8; efficacy: PR | 2 points for skin, 1 point for eye, 1 point for oral cavity, 1 point for lower digestive tract, 3 points for lung (17%), 1 point for joint fascia, comprehensive score 8; efficacy: PR | 2 points for skin, 2 points for eye, 1 point for oral cavity, 1 point for lower digestive tract, 3 points for lung (15.9%), 1 point for joint fascia, comprehensive score 8; efficacy: PR | 0 points for skin, 2 points for eye, 0 points for oral cavity, 0 points for lower digestive tract, 3 points for lung (17.6%), 0 points for joint fascia, comprehensive score 6; efficacy: PR | - |
| 6 | Cyclosporine soft capsule + prednisone tablet | 15mg bid | C8 | 1 point for skin, 2 points for eye, 1 point for oral cavity, 3 points for lung (27.6%), 1 point for joint fascia, comprehensive score 7 | 1 point for skin, 2 points for eye, 1 point for oral cavity, 3 points for lung (28.7%), 1 point for joint fascia, comprehensive score 6; efficacy: SD | 1 point for skin, 2 points for eye, 0 points for oral cavity, 3 points for lung (27.8%), 1 point for joint fascia, comprehensive score 5; efficacy: PR | 1 point for skin, 2 points for eye, 1 point for oral cavity, 3 points for lung (28.4%), 0 points for joint fascia, comprehensive score 4; efficacy: PR | - | - |
| 7 | Tacrolimus + prednisone tablet + mycophenolate mofetil + sorafenib | 15mg bid | C8 | 2 points for skin, 2 points for eye, 2 points for oral cavity, 1 point for esophagus, comprehensive score 4 | 2 points for skin, 2 points for eye, 1 point for oral cavity, 0 points for esophagus, comprehensive score 3; efficacy: PR | 1 point for skin, 2 points for eye, 1 point for oral cavity, 0 points for esophagus, comprehensive score 2; efficacy: PR | 1 point for skin, 2 points for eye, 1 point for oral cavity, 0 points for esophagus, comprehensive score 2; efficacy: PR | - | - |
| 8 | Tacrolimus + prednisone tablet + mycophenolate mofetil | 15mg bid | C7 | 3 points for skin, 1 point for eye, 3 points for oral cavity, 2 points for esophagus, 2 points for joint fascia, 2 points for lung (48%), comprehensive score 8 | 3 points for skin, 1 point for eye, 3 points for oral cavity, 2 points for esophagus, 2 points for joint fascia, 2 points for lung (55%), comprehensive score 7; efficacy: SD | 3 points for skin, 1 point for eye, 3 points for oral cavity, 2 points for esophagus, 2 points for joint fascia, 2 points for lung (55%), comprehensive score 7; efficacy: SD | 2 points for skin, 0 points for eye, 2 points for oral cavity, 1 point for esophagus, 2 points for joint fascia, 2 points for lung (52.7%), comprehensive score 6; efficacy: PR | - | - |

| | | | | | | | | | |
|---|---|---|---|---|---|---|---|---|---|
| Note: "--" indicates that the data has not been obtained yet. | | | | | | | | | |

## Claims

1. A compound of formula **I,** a stereoisomer thereof or a pharmaceutically acceptable salt thereof for use in treating chronic graft-versus-host disease in a patient:

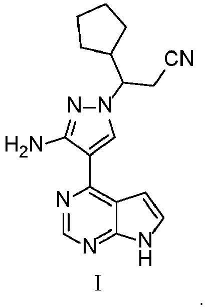

2. A pharmaceutical composition for use in treating chronic graft-versus-host disease in a patient, comprising the compound of formula **I,** the stereoisomer thereof or the pharmaceutically acceptable salt thereof for the use according to claim 1.

3. Use of the compound of formula **I,** the stereoisomer thereof or the pharmaceutically acceptable salt thereof for the use according to claim 1, or the pharmaceutical composition according to claim 2 for preparing a medicament for treating chronic graft-versus-host disease in a patient.

4. The use according to claim 3, wherein the chronic graft-versus-host disease refers to a complication caused by allogeneic stem cell transplantation.

5. The use according to claim 3 or 4, wherein the chronic graft-versus-host disease includes classical chronic graft-versus-host disease and acute and chronic overlap syndrome.

6. The use according to any one of claims 3-5, wherein the chronic graft-versus-host disease is selected from the group consisting of mild, moderate and severe chronic graft-versus-host diseases.

7. The use according to any one of claims 3-6, wherein for the mild chronic graft-versus-host disease, there are 1 or 2 organs or sites involved (except lung) (score ≤ 1); for the moderate, there is at least 1 organ or site involved (score = 2), or 3 or more organs or sites involved (score ≤ 1), or lung involved (score = 1); for the severe, there is any organ or site involved reaching a score of 3 or lung involved (score ≥ 2).

8. The use according to any one of claims 3-7, wherein the chronic graft-versus-host disease is refractory and/or dependent chronic graft-versus-host disease.

9. The use according to any one of claims 3-8, wherein the chronic graft-versus-host disease is glucocorticoid-refractory and/or dependent chronic graft-versus-host disease.

10. The use according to claim 9, wherein the glucocorticoid is selected from the group consisting of prednisone, meprednisone, prednisone acetate, prednisolone, methylprednisolone, prednisolone acetate, prednisolone sodium succinate, methylprednisolone sodium succinate, betamethasone, beclomethasone propionate, hydrocortisone, dexamethasone, salmeterol/fluticasone, and pulsed high-dose glucocorticoid.

11. The use according to any one of claims 3-10, wherein a daily dose of the compound of formula **I,** the stereoisomer thereof, or the pharmaceutically acceptable salt thereof is 1 mg to 50 mg, preferably 5 mg to 30 mg, 5 mg to 25 mg, 5 mg to 20 mg, and 10 mg to 20 mg.

12. The use according to any one of claims 3-11, wherein the compound of formula **I,** the stereoisomer thereof, or the pharmaceutically acceptable salt thereof is administered once or multiple times a day.

13. The use according to any one of claims 3-12, wherein the compound of formula **I,** the stereoisomer thereof, or the pharmaceutically acceptable salt thereof is administered in a single-dose or a multiple-dose once or twice a day.

14. The use according to any one of claims 3-13, wherein 28 days constitute one treatment cycle, in which the compound of formula **I,** the stereoisomer thereof or the pharmaceutically acceptable salt thereof, or the pharmaceutical composition is administered consecutively from day 1 to day 28.

15. The use according to any one of claims 3-14, wherein 28 days constitute one treatment cycle, in which a total dose of 140-840 mg of the compound of formula **I,** the stereoisomer thereof or the pharmaceutically acceptable salt thereof, or the pharmaceutical composition is administered calculated based on the free base form of the compound of formula **I.**
